# EUROPEAN PATENT APPLICATION

(11) **EP 2 210 893 A1**
(43) Date of publication of application: **28.07.2010**
(21) Application number: 10004589.7
(22) Date of filing: 04.11.2004
(51) Int. Cl.: C07D 498/22, A61K 31/437, A61P 31/04

(54) **Polymorphous forms of rifaximin as antibiotics**

(30) Priority: 17.11.2003 IT MI20032144
(62) Divisional of application: 04797615.4
(71) Applicant: ALFA WASSERMANN S.p.A., 65020 Alanno Scalo (Pescara) (IT)
(72) Inventor: The designation of the inventor has not yet been filed
(74) Representative: Hiebl, Inge Elisabeth

(57) **Abstract**

Crystalline polymorphic forms of rifaximin (INN), referred to as rifaximin α and rifaximin β, and a poorly crystalline form referred to as rifaximin γ, useful in the production of medicaments containing rifaximin for the oral and topical use and obtained by means of a crystallization process carried out by hot-dissolving the raw rifaximin in ethyl alcohol and causing the crystallization of the product by addition of water at a fixed temperature and for a fixed period of time, followed by drying under controlled conditions until reaching a precise water content in the end product, are the object of the invention.

## Description

The present invention relates to rifaximin polymorphic forms α, β and γ, the processes for their preparation and the use thereof in the manufacture of medicinal preparations for the oral or topical route.

### Background of the invention

Rifaximin (INN; see The Merck Index, XIII Ed., 8304) is an antibiotic belonging to the rifamycin class, namely a pyrido-imidazo rifamycin described and claimed in Italian Patent IT 1154655, while EP 0161534 discloses and claims a process for its production starting from rifamycin O (The Merck Index, XIII Ed., 8301).

Both these patents generically describe the purification of rifaximin by crystallization in suitable solvents or solvent systems and summarily show in some examples that the resulting product can be crystallized from the 7:3 mixture of ethyl alcohol/water and dried both under atmospheric pressure and under vacuum. Neither information concerning the experimental conditions of crystallization and drying, nor any distinctive crystallographic characteristic of the obtained product are reported.

The presence of different polymorphs had not been ascertained and therefore the experimental conditions described in both patents had been developed with the aim of obtaining a homogeneous product having suitable purity from the chemical point of view, apart from the crystallographic aspects of the product itself.

It has now been unexpectedly found that some polymorphic forms of rifaximin exist whose formation depends on the solvent as well as on the conditions of time and temperature at which both crystallization and drying are carried out.

In the present application, these polymorphic forms will be hereinafter referred to as rifaximin α (figure 1) and rifaximin β (figure 2) on the basis of their respective specific diffractograms, while the poorly crystalline form with a high content of amorphous component will be identified as rifaximin γ (figure 3).

The rifaximin polymorphic forms have been characterized through the powder X-ray diffraction technique.

The identification and characterization of these polymorphic forms and, contemporarily, the definition of the experimental conditions for obtaining them is very important for a compound endowed with pharmacological activity which, like rifaximin, is marketed as medicinal preparation, both for human and veterinary use. It is in fact known that the polymorphism of a compound that can be used as active principle contained in a medicinal preparation can influence the pharmaco-toxicologic properties of the drug. Different polymorphic forms of an active principle administered as drug in the oral or topical form can modify many properties thereof, like bioavailability, solubility, stability, colour, compressibility, flowability and workability with consequent modification of the profiles of toxicological safety, clinical effectiveness and productive efficiency.

What above mentioned is confirmed by the fact that the authorities that regulate the grant of the authorization for the admission of the drugs on the market require that the manufacturing methods of the active principles be standardized and controlled in such a way as to give homogeneous and sound results in terms of polymorphism of the production batches (CPMP/QWP/96, 2003 - Note for Guidance on Chemistry of new Active Substance; CPMP/ICH/367/96 - Note for guidance specifications: test procedures and acceptance criteria for new drug substances and new drug products: chemical substances; Date for coming into operation: May 2000).

The need for the above-mentioned standardization has further been strengthened in the field of rifamycin antibiotics by Henwood S.Q., de Villiers M.M., Liebenberg W. and Lötter A.P., Drug Development and Industrial Pharmacy, 26 (4), 403-408, (2000), who have ascertained that different production batches of rifampicin (INN) prepared by different manufacturers differ among them because they show different polymorphy characteristics, and as a consequence they show different dissolution profiles together with consequent alteration of the respective pharmacological properties.

Following the processes of crystallization and drying generically disclosed in IT 1154655 and EP 0161534 it has been found that under some experimental conditions the rifaximin poorly crystalline form is obtained, while under other experimental conditions the other crystalline polymorphic forms of rifaximin are obtained. Moreover it has been found that some parameters, absolutely not disclosed in the above-mentioned patents, for instance the conditions of preservation and the relative humidity of the ambient, have the surprising effect to determine the form of the polymorph.

The rifaximin polymorphic forms object of the present application have never been found or hypothesized, as it was generally assumed that a sole homogeneous product would always have been obtained, whichever the method chosen within the range of the described conditions, irrespective of the conditions used for crystallizing, drying and preserving.

It has now been found that the formation of the α, β and γ forms depends on the presence of water in the crystallization solvent, on the temperature at which the product is crystallized and on the amount of water present in the product at the end of the drying phase.

The form α, the form β and the form γ of rifaximin have then been synthesised and they are the object of the invention.

Moreover it has been found that the presence of water in rifaximin at the solid state is reversible, so that absorption and/or cession of water can take place in time in the presence of suitable ambient conditions; consequently rifaximin is susceptible of transition from one form to another, also remaining in the solid state, without need to be again dissolved and crystallized. For instance when polymorph α absorbs hydration water until a content higher than 4.5%, it changes into polymorph β, which in its turn changes into polymorph α upon drying until a water content lower than 4.5%.

These results have a remarkable importance as they determine the conditions of industrial manufacturing of some working steps, which could not be considered critical for the determination of the polymorphism of a product, for instance the washing of a crystallized product, the conditions of preservation of the end product, or the characteristics of the container in which the product is preserved.

Rifaximin exerts its broad antibacterial activity in the gastrointestinal tract against localized gastrointestinal bacteria that cause infectious diarrhea, including anaerobic strains. It has been reported that rifaximin is characterized by a negligible systemic absorption, due to its chemical and physical characteristics *(*Descombe J.J. et al. Pharmacokinetic study of rifaximin after oral administration in healthy volunteers. Int J Clin Pharmacol Res, 14 (2), 51-56, (1994*)*).

Now we have found that the level of rifaximin systemic adsorption can be modulated, and this is part of the present invention, by administering distinct polymorphic forms of rifaximin, namely rifaximin α, rifaximin β and rifaximin γ. It is possible to have a difference in the adsorption of almost 600 folds in the range from 0.001 to 1 µg/ml in blood.

The evidenced difference in the bioavailability is important because it can differentiate the pharmacological and toxicological behaviour of the three polymorphous of rifaximins α, β and γ.

As a matter of fact, rifaximin α and rifaximin β are negligibly absorbed through the oral route, while rifaximin γ shows a mild absorption.

Rifaximins α and β, which are practically not absorbed, might act only through a topical action, including the case of the gastro-intestinal tract, with the advantage of very low toxicity.

On the other hand, rifaximin γ, which is mildly absorbed, can find an advantageous use against systemic microorganisms, able to hide themselves and to partially elude the action of the topic antibiotics.

In respect of possible adverse events coupled to the therapeutic use of rifaximin of particular relevance is the induction of bacterial resistance to the antibiotics.

Generally speaking, in therapeutic practice with antibiotics bacterial resistance to the same or other antibiotics can occur when resistant strains arise. In the case of rifaximin, this aspect is particularly relevant, since rifaximin belongs to the rifamycin family, a member of which, rifampicin, is largely used in the treatment of tuberculosis. The current short course treatment of tuberculosis is a combination therapy involving four active pharmaceutical ingredients: rifampicin, isoniazid, ethambutol and pyrazinamide, rifampicin playing a pivotal role. Therefore, any drug which jeopardized the efficacy of the therapy by selecting for resistance to rifampicin would be harmful. (Kremer L. et al. "Re-emergence of tuberculosis: strategies and treatment ", Expert Opin.Investig.Drugs, 11 (2), 153-157, (2002*)).*

In principle, considering the structural similarity between rifaximin and rifampicin, the use of rifaximin might induce the selection resistant strains of *M. tuberculosis* and cross-resistance to rifampicin. In order to avoid this negative event it is crucial to have a control of the quantity of systemically absorbed rifaximin.

From this point of view, the difference found in the systemic absorption of the α , β and γ forms of rifaximin is significant, since also at sub-inhibitory concentration of rifaximin, such as in the range from 0.1 to 1 µg/ml, selection of resistant mutants has been demonstrated to be possible (Marchese A. et al. In vitro activity of rifaximin, metronidazole and vancomycin against clostridium difficile and the rate of selection of spontaneously resistant mutants against representative anaerobic and aerobic bacteria, including ammonia-producing species. Chemotherapy, 46(4), 253-266, (2000*))*.

What stated above clearly shows the importance of the rifaximin polymorphic forms of the present invention, and of the various industrial processes of the invention for the preparation of pure single forms having different pharmacological properties.

According to what stated above, the importance of the present invention, which has led to the knowledge of the existence of the above mentioned rifaximin polymorphic forms and to various industrial routes for manufacturing pure single forms having different pharmacological properties, is clearly strengthened.

The above-mentioned α, β and γ forms can be advantageously used as pure and homogeneous products in the manufacture of medicinal preparations containing rifaximin.

As already stated, the process for industrial preparation rifaximin from rifamycin O disclosed and claimed in EP 0161534 is lacking from the standpoints of purification and identification of the product obtained; it shows some limits also from the synthetic point of view as regards, for instance, the very long reaction times, from 16 to 72 hours, which are poorly suited to an industrial use, and moreover because it does not provide for the in situ reduction of the rifaximin oxidized that may be formed in the reaction mixture.

Therefore, a further object of the present invention is an improved process for the industrial preparation of the α, β and γ forms of rifaximin herein claimed as products and usable as defined and homogeneous active principles in the manufacture of the medicinal preparations containing such active principle.

### Description of the invention

The present invention relates to the form α, the form β and the form γ of the antibiotic known as rifaximin (INN), the processes for their preparation and the use thereof in the manufacture of medicinal preparations for the oral or topical route.

The process of the present invention comprises reacting one molar equivalent of rifamycin O with an excess of 2-amino-4-methylpyridine, preferably from 2.0 to 3.5 molar equivalents, in a solvent mixture consisting of water and ethyl alcohol in volumetric ratios between 1:1 and 2:1, for a time between 2 and 8 hours at a temperature between 40°C and 60°C.

After completion of the reaction, the reaction mass is cooled to room temperature and added with a solution of ascorbic acid in a mixture of water, ethyl alcohol and aqueous concentrated hydrochloric acid, under strong stirring, in order to reduce the small amount of oxidized rifaximin that forms during the reaction. Finally the pH is adjusted to about 2.0 by further addition of hydrochloric acid concentrated aqueous solution, in order to better remove the excess of 2-amino-4-methylpyridine used in the reaction. The suspension is filtered and the resulting solid is washed with the same solvent mixture water/ethyl alcohol as used in the reaction. Such semifinished product is called "raw rifaximin".

The raw rifaximin can be directly submitted to the subsequent purification step. Alternately, in case long times of preservation of the semifinished product are expected, the raw rifaximin can be dried under vacuum at a temperature lower than 65°C for a time between 6 and 24 hours, such semifinished product is called "dried raw rifaximin".

The resulting raw rifaximin and/or dried raw rifaximin are purified by dissolution in ethyl alcohol at a temperature between 45°C and 65°C, followed by crystallization by addition of water, preferably in weight amounts between 15% and 70% to the weight amount of ethyl alcohol used for the dissolution, and by keeping the resulting suspension at a temperature between 50°C and 0°C under stirring during a time between 4 and 36 hours.

The suspension is filtered and the obtained solid is washed with water and dried under vacuum or under normal pressure, optionally in the presence of a drying agent, at a temperature between room temperature and 105°C for a time between 2 and 72 hours.

The achievement of the α, β and γ forms depends on the conditions selected for the crystallization. In particular, the composition of the solvent mixture used for the crystallization, the temperature at which the reaction mixture is kept after the crystallization and the period of time at which that temperature is kept, have proven to be critical.

More precisely, rifaximin γ is obtained when the solution is brought to a temperature between 28°C and 32°C to start precipitation and the resulting suspension is further cooled to 0°C and kept at this temperature for a time between 6 and 24 hours.

The suspension is filtered, the solid is washed with demineralized water and is dried to a water content between 1.0% and 2.0%.

The α and β rifaximins are obtained when the temperature is first brought to a value between 28°C and 32°C in order to start crystallization, then the suspension is brought to a temperature between 40°C and 50°C and kept at this value for a time between 6 and 24 hours, then the suspension is quickly cooled to 0°C in 15 minutes to one hour, then is filtered, the solid is washed with water and then dried.

The drying steps plays an important role in obtaining the rifaximin α and β polymorphic forms and has to be monitored by a method suited to water dosage, such as the Karl Fischer method, in order to check the amount of remaining water present in the product under drying.

Rifaximin α or rifaximin β are obtained by drying to different final water contents, be they higher or lower than 4.5%, and do not depend on the experimental conditions of pressure and temperature at which such critical water contents are achieved. In fact, the two polymorphic forms, with higher or lower water content, can be obtained by drying under vacuum or at atmospheric pressure, at room temperature or at high temperatures, optionally in the presence of drying agents, provided that the drying is prolonged for the time necessary to reach the water content characteristic for each polymorphic form.

The polymorphic form β is obtained when the drying of the product crystallized and washed with water is stopped at water contents higher than 4.5%, measured by Karl Fischer, preferably between 5.0% and 6.0%, while the polymorphic form α is obtained when drying is continued until water contents lower than 4.5%, preferably between 2.0% and 3.0%,.

Both the form γ and the forms α and β of rifaximin are hygroscopic, they reversibly absorb water in time in the presence of suitable environmental conditions of pressure and humidity and are susceptible of transformation from one form into another.

When the polymorphic form α is kept under conditions of relative humidity higher than 50% for a time between 12 and 48 hours, it changes into the polymorphic form β, which in its turn is transformed into the polymorphic form α upon drying to a water content lower than 4.5%, preferably comprised between 2.0% and 3.0%,.

Another type of transition exists between the form γ and the forms α and β, depending upon the temperatures kept during the phase of precipitation of rifaximin.

In particular, the form γ turns into the forms α or β when a suspension of the form γ of rifaximin is kept in an ethyl alcohol/water 7:3 (V/V) solvent mixture at a temperature between 38°C and 50°C under strong stirring for a prolonged time, preferably comprised between 6 and 36 hours.

After filtration and washing with demineralized water, drying to a water content higher than 4.5%, preferably between 5.0% and 6.0%, affords the polymorphic form β, while when drying is continued to a water content lower than 4.5%, preferably between 2.0% and 3.0%, gives the form α.

Rifaximins α and β can in their turn change into rifaximin γ by dissolution in ethyl alcohol and treatment of the resulting solution as previously described for the preparation of the form γ.

These transitions from one form into another are very important for the invention, as they can be provide an alternative process for the preparation of the form desired for the production of the medicinal preparations. Therefore, the process that allows to transform rifaximin γ into rifaximin α or β in a valid industrial manner, the process that allows to transform rifaximin α or β into rifaximin γ in a valid industrial manner, the process that allows to transform rifaximin α into rifaximin β in a valid industrial manner or *vice versa* rifaximin β into rifaximin α, are important parts of the invention.

The process concerning the transformation of rifaximin γ into rifaximin α or rifaximin β comprises suspending rifaximin γ in a solvent mixture consisting of ethyl alcohol/water in 7:3 volumetric ratio, warming the suspension to a temperature between 38°C and 50°C and keeping it at this temperature under strong stirring for a time between 6 and 36 hours. The suspension is then filtered, the solid is washed with water and dried; the polymorphic form β is obtained when drying is carried out to a water content between 5.0% and 6.0% measured by the Karl Fischer method, while the polymorphic form α is obtained when drying is continued to a water content between 2.0% and 3.0%.

The process for the preparation of the form γ starting from rifaximin α or β comprises dissolving the α or β form in ethyl alcohol under stirring, at a temperature between 50°C and 60°C, adding demineralized water to an ethyl alcohol/water 7:3 volumetric ratio, cooling the solution to 30°C under strong stirring, cooling the precipitate to 0°C and keeping the suspension under stirring at 0°C for a time between 6 and 24 hours. The suspension is then filtered, the solid is washed with water and dried to a water content lower than 2.0% thereby obtaining rifaximin γ.

The process for the transformation of the form α into the form β consists in keeping powder rifaximin α in an ambient having relative humidity higher than 50% for the time required to obtain a water content in the powder higher than 4.5%, which time is usually between 12 and 48 hours.

The process for the transformation of the form β into the form α consists in drying powder rifaximin β under vacuum or under conditions of normal pressure, optionally in the presence of a drying agent, at a temperature between the room temperature and 105°C, for a time between 2 and 72 hours, to obtain a water content in the powder lower than 4.5%, preferably between 2.0% and 3.0%.

It is evident from what stated above that during preservation of the product particular care should be taken so that the ambient conditions do not affect the water content of the product, by preserving the product in an environment having controlled humidity or in closed containers that allow no significant exchanges of water with the exterior.

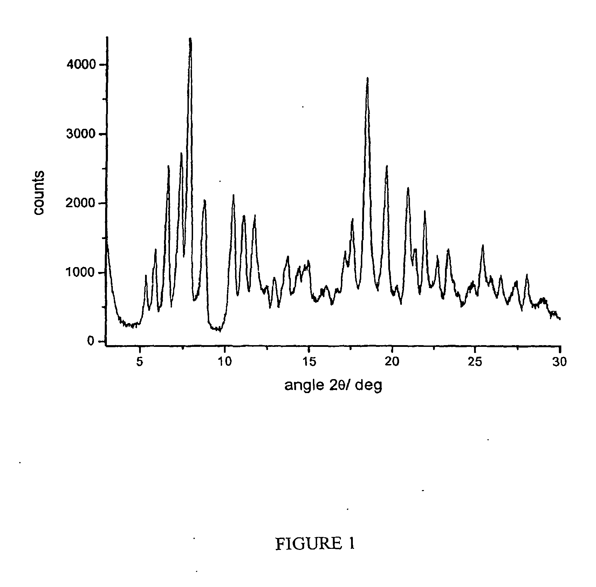

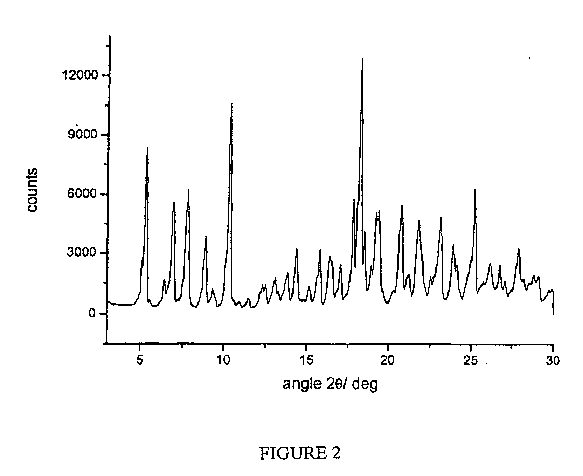

The rifaximin α polymorph is characterised by a water content lower than 4.5%, preferably between 2.0% and 3.0% and by a powder X-ray diffractogram (reported in figure 1) which shows peaks at the values of the diffraction angles 2θ of 6.6°; 7.4°; 7.9°; 8.8°; 10.5°; 11.1°; 11.8°; 12.9°; 17.6°; 18.5°; 19.7°; 21.0°; 21.4°; 22.1°. The rifaximin β polymorph is characterised by a water content higher than 4.5%, preferably between 5.0% and 6.0%, and by a powder X-ray diffractogram (reported in figure 2) which shows peaks at the values of the diffraction angles 2θ of 5.4°; 6.4°; 7.0°; 7.8°; 9.0°; 10.4°; 13.1°; 14.4°; 17.1°; 17.9°; 18.3°; 20.9°.

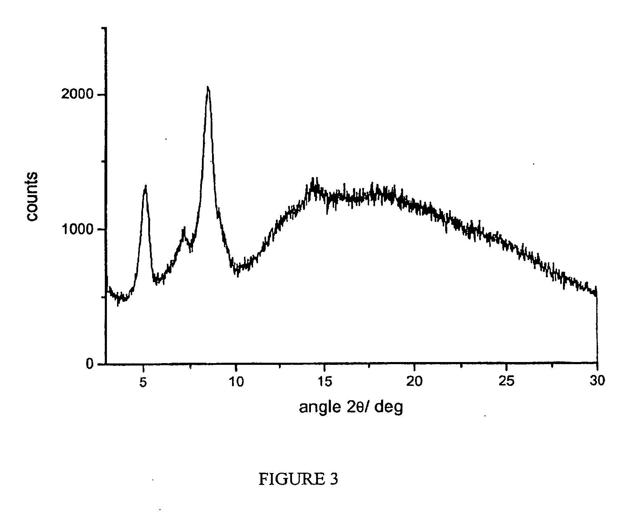

The rifaximin γ polymorph is characterised by a powder X-ray diffractogram much poorer because of the poor crystallinity; the significant peaks are at the values of the diffraction angles 2θ of 5.0°; 7.1 °; 8.4° as reported in figure 3.

The diffractograms have been carried out using a Philips X'Pert instrument fitted with Bragg-Brentano geometry and under the following working conditions:
X-ray tube: **Copper**
Radiation used: **K (**α**1), K (**α**2)**
Tension and current of the generator: **KV 40, mA 40**
Monocromator: **Graphite**
Step size: **0.02**
Time per step: **1.25 seconds**
Starting and final angular 2θ value: **3.0°÷30.0°**

The evaluation of the water content in the analyzed samples has always been carried out by means of the Karl Fischer method.

Rifaximin α, rifaximin β and rifaximin γ significantly differ from each from other also in terms of bioavailability and intrinsic dissolution.

A bioavailability study of the three polymorphs has been carried out on Beagle female dogs, by feeding them orally with a dose of 100 mg/kg of one of the polymorphs, collecting blood samples from the jugular vein of each animal before each dosing and 1, 2, 4, 6, 8 and 24 hours after each dosing, transferring the samples into tubes containing heparin and separating the plasma by centrifugation.

The plasma has been assayed for rifaximin on the validated LC-MS/MS (Liquid Chromathography-Mass Spectrometry/Mass Spectrometry) method and the maximum plasma concentration observed (Cₘₐₓ), the time to reach the (Cₘₐₓ), (tₘₐₓ), and the area under the concentration - time curve (AUC) have been calculated.

The experimental data reported in the following table 1 clearly show that rifaximin α and rifaximin β are negligibly absorbed, while rifaximin γ is absorbed at a value (Cₘₐₓ = 0.668 µg/ml) comprised in the range of from 0.1 to 1.0 µg/ml.

**Table 1**

| **Pharmacokinetic parameters for rifaximin polymorphs following single oral administration of 100 mg/kg by capsules to female dogs.** | | | |
|---|---|---|---|
| | **Cmax ng/ml** | **tₘₐₓ h** | **AUC₀₋₂₄ ng·h /ml** |
| | **Mean** | **Mean** | **Mean** |
| **Polimorph** α | 2.632 | 9.5 | 13 |
| **Polimorph** β | 1.096 | 4 | 11 |
| **Polimorph** γ | 668.22 | 2.25 | 3908 |

Intrinsic dissolution tests have been carried out on each of the three polymorphs according to the method described in the monograph 1087 at pages 2512 - 2513 of the USP (U.S. Pharmacopoeia) 27, clearly showing significant differences among rifaximin α, rifaximin β and rifaximin γ.

A sample of each rifaximin polymorph has been put into a die and compressed at 5 tons by means of a punch of a hydraulic press to obtain a compacted pellet.

The die-holder containing the compacted pellet has then been mounted on a laboratory stirring device, immersed in a dissolution medium and rotated by means of the stirring device.

The test, carried out in a dissolution medium made of aqueous phosphate buffer at ph 7.4 and of sodium lauryl sulfate at a temperature of 37±0.5°C, has shown significant differences among the instrinsic dissolution rates exihibited by the three polymorphs.

Rifaximin α has shown disintegration of the compacted pellet within 10 minutes so that it has not been possible to calculate the value of its intrinsic dissolution, while the intrinsic dissolution of rifaximin γ has been about ten times as much that of rifaximin β in accordance with its bioavailability which is more than hundred times as much that of rifaximin β.

The above experimental results further point out the differences existing among the three rifaximin polymorphs.

The forms α, β and γ can be advantageously used in the production of medicinal preparations having antibiotic activity, containing rifaximin, for both oral and topical use. The medicinal preparations for oral use will contain rifaximin α or β or γ together with the usual excipients, for example diluting agents such as mannitol, lactose and sorbitol; binding agents such as starchs, gelatines, sugars, cellulose derivatives, natural gums and polyvinylpyrrolidone; lubricating agents such as talc, stearates, hydrogenated vegetable oils, polyethylenglycol and colloidal silicon dioxide; disintegrating agents such as starchs, celluloses, alginates, gums and reticulated polymers; coloring, flavoring and sweetening agents.

The present invention relates to all of the solid preparations administrable by the oral route, for instance coated and uncoated tablets, of soft and hard gelatine capsules, sugar-coated pills, lozenges, wafer sheets, pellets and powders in sealed packets.

The medicinal preparations for topical use will contain rifaximin α or β or γ together with usual excipients, such as white petrolatum, white wax, lanoline and derivatives thereof, stearylic alcohol, propylene glycol, sodium lauryl sulfate, ethers of fatty polyoxyethylene alcohols, esters of fatty polyoxyethylene acids, sorbitan monostearate, glyceryl monostearate, propylene glycol monostearate, polyethylene glycols, methylcellulose, hydroxymethyl propylcellulose, sodium carboxymethylcellulose, colloidal aluminium and magnesium silicate, sodium alginate.

The present invention relates to all of the topical preparations, for instance ointments, pomades, creams, gels and lotions.

Further disclosed are the following items:
1. Polymorph α, of rifaximin characterized by a water content lower than 4.5%, preferably comprised between 2.0% and 3.0%, and by a powder X-ray diffractogram showing peaks at values of the diffraction angles 2θ of 6.6°; 7.4°; 7.9°; 8.8°; 10.5°; 11.1°; 11.8°; 12.9°; 17.6°; 18.5°; 19.7°; 21.0°; 21.4°; 22.1°.
2. Polymorph β of rifaximin characterized by a water content higher than 4.5%, preferably comprised between 5.0% and 6.0% and by a powder X-ray diffractogram showing peaks at values of the diffraction angles 2θ of 5.4°; 6.4°; 7.0°; 7.8°; 9.0°; 10.4°; 13.1°, 14.4°; 17.1°; 17.9°; 18.3°; 20.9°.
3. Polymorph γ of rifaximin characterized by a water content comprised between 1.0% and 2.0% and by a powder X-ray diffractogram showing a mainly amorphous profile and few significant peaks at values of diffraction angles 2θ of 5.0°; 7.1°; 8.4°.
4. A process for the production of rifaximins α, β and γ, **characterized in that** a molar equivalent of rifamycin O is reacted with an excess of 2-amino-4-methylpyridine, preferably from 2.0 to 3.5 molar equivalents, in a solvent mixture consisting of water and ethyl alcohol in volumetric ratios between 1:1 and 2:1 for a time between 2 and 8 hours, at a temperature between 40°C and 60°C; the reaction mixture is treated at room temperature with a solution of ascorbic acid in a mixture of water, ethyl alcohol and concentrated aqueous hydrochloric acid, then brought to pH 2.0 by hydrochloric acid concentrated aqueous solution, the suspension is filtered, the resulting solid is washed with the same water/ethyl alcohol solvent mixture as used in the previous reaction, the resulting raw rifaximin is purified by dissolution in ethyl alcohol at a temperature between 45°C and 65°C; precipitation by addition of water, preferably in weight amounts between 15% and 70% to the weight amount of ethyl alcohol used for the dissolution; lowering of the temperature of the suspension to values between 50°C and 0°C under stirring for a time between 4 and 36 hours, and finally filtration of the suspension, washing the resulting solid with water and drying it under vacuum or under conditions of normal pressure, optionally in the presence of a drying agent, at a temperature between room temperature and 105°C, for a time between 2 and 72 hours.
5. A process according to item 4 for the preparation of rifaximin α, **characterized in that**, after addition of water to the raw rifaximin ethanol solution, temperature is lowered to a value between 28°C and 32°C to start crystallization, the resulting suspension is kept between 40°C and 50°C under stirring for a time between 6 and 24 hours, cooled to 0°C during a time between 15 minutes and one hour, then filtered, and the resulting solid is dried to a water content lower than 4.5%, preferably between 2.0% and 3.0%.
6. A process according to item 4 for the preparation of rifaximin β, **characterized in that**, after addition of water to the raw rifaximin ethanol solution, temperature is lowered to a value between 28°C and 32°C to start crystallization, the resulting suspension is kept between 40°C and 50°C under stirring for a time between 6 and 24 hours, cooled to 0°C during a time between 15 minutes and one hour, then filtered, and the resulting solid is dried to a water content higher than 4.5%, preferably between 5.0% and 6.0%.
7. A process according to item 4 for the preparation of rifaximin γ **characterized in that**, after addition of water to the raw rifaximin ethanol solution, temperature is lowered to a value between 28°C and 32°C to start crystallization, the resulting suspension is cooled to about 0°C and kept at this temperature, under stirring, for a time between 6 and 24 hours, then is filtered, and the resulting solid is dried to a water content between 1.0% and 2.0%.
8. A process for the preparation of rifaximin α, **characterized in that** rifaximin γ is suspended in a solvent mixture consisting of ethyl alcohol/water in a 7:3 volumetric ratio, the suspension is heated to a temperature between 38°C and 50°C and kept at this temperature, under stirring, for a time between 6 and 36 hours, the suspension is filtered, the resulting solid is washed with water and dried to a water content lower than 4.5%, preferably between 2.0% and 3.0%.
9. A process for the preparation of rifaximin β, **characterized in that** rifaximin γ is suspended in a solvent mixture consisting of ethyl alcohol/water in a 7:3 volumetric ratio, the suspension is heated to a temperature between 38°C and 50°C and kept at this temperature, under stirring, for a time between 6 and 36 hours, the suspension is filtered, the resulting solid is washed with water and is dried to a water content higher than 4.5%, preferably between 5.0% and 6.0%.
10. A process for the preparation of rifaximin γ, **characterized in that** rifaximin α or β is dissolved in ethyl alcohol at a temperature between 50°C and 60°C, demineralized water is added to an ethyl alcohol/water 7:3 volumetric ratio, the solution is cooled to 30°C under strong stirring and the resulting suspension is cooled to 0°C and kept at this temperature for a time between 6 and 24 hours, then it is filtered and the resulting solid is washed with water and dried to a water content lower than 2.0%.
11. A process for the preparation of rifaximin β **characterized in that** rifaximin α is kept in an ambient having relative humidity higher than 50% for a time between 12 and 48 hours.
12. A process for the preparation of rifaximin α **characterized in that** rifaximin β is dried under atmospheric pressure, or under vacuum, or in the presence of a drying agent, at a temperature between the room temperature and 105°C, for a time between 2 and 72 hours.
13. The use of rifaximin α in the preparation of medicaments for oral use with antibiotic activity together with the usual excipients like diluting, binding, lubricating, disintegrating, coloring, flavoring and sweetening agents.
14. The use of rifaximin β in the preparation of medicaments for the oral use having antibiotic activity, together with conventional excipients.
15. The use of rifaximin γ in the preparation of medicaments for the oral use having antibiotic activity, together with conventional excipients.
16. The use according to items 13 to 15 **characterized in that** the preparations for oral use are selected from coated and uncoated tablets, hard and soft gelatine capsules, sugar-coated pills, lozenges, wafer sheets, pellets and powders in sealed packet.
17. The use of rifaximin α in the preparation of medicaments with antibiotic activity for topical use.
18. The use of rifaximin β in the preparation of medicaments with antibiotic activity for topical use.
19. The use of rifaximin γ in the preparation of medicaments with antibiotic activity for topical use.
20. The use according to items 17 to 19 **characterized in that** the preparations for the topical use are selected from ointments, pomades, creams, gels and lotions.

The invention is further illustrated by some examples. Such examples are not to be taken as a limitation of the invention, it is in fact evident that the α, β and γ forms can be obtained by suitably combining between them the above mentioned conditions of crystallization and drying.

### Example 1

### Preparation of raw rifaximin and of dried raw rifaximin

In a three-necked flask equipped with mechanic stirrer, thermometer and reflux condenser, 120 ml of demineralized water, 96 ml of ethyl alcohol, 63.5 g of rifamycin O and 27.2 g of 2-amino-4-methylpyridine are loaded in succession at room temperature. After loading, the mass is heated at 47±3°C and kept under stirring at this temperature for 5 hours, then is cooled to 20±3°C and, during 30 minutes, is added with a mixture, prepared separately, of 9 ml of demineralized water, 12.6 ml of ethyl alcohol, 1.68 g of ascorbic acid and 9.28 g of aqueous concentrated hydrochloric acid. After completion of the addition, the mass is kept under stirring for 30 minutes at an inner temperature of 20±3°C then 7.72 g of concentrated hydrochloric acid are dripped until a pH equal to 2.0, while keeping said temperature.

After completion of the addition, the mass is kept under stirring for 30 minutes, keeping an inner temperature of 20°C, then the precipitate is filtered and washed with a mixture of 32 ml of demineralized water and of 25 ml of ethyl alcohol. The resulting "raw rifaximin" (89.2 g) is dried under vacuum at room temperature for 12 hours obtaining 64.4 g of "dried raw rifaximin" which shows a water content of 5.6% and a diffractogram corresponding to the polymorphic form β. The product is further dried under vacuum until constant weight to afford 62.2 g of dried raw rifaximin having a water content of 2.2%, whose diffractogram corresponds to the polymorphic form α.

The product is hygroscopic and the obtained polymorphic form is reversible: the polymorphic form α absorbs water from atmospheric humidity, depending on the relative humidity and the exposure time. When the water content absorbed by the polymorphic form α becomes higher than 4.5%, polymorphous α turns to polymorphous β. This in its turn loses part of water by drying, changing into the polymorphic form α when a water content between 2.0% and 3.0% is reached.

### Example 2

### Preparation of rifaximin γ

163 ml of ethyl alcohol and 62.2 g of dried raw rifaximin are loaded at room temperature into a three-necked flask equipped with mechanic stirrer, thermometer and reflux condenser. The suspension is heated at 57±3°C under stirring until complete dissolution of the solid, and added with 70 ml of demineralized water at this temperature in 30 minutes. After completion of the addition the temperature is brought to 30°C in 40 minutes and kept at this value until complete crystallization, then the temperature is further lowered to 0°C in 2 hours and kept at this value for 6 hours. The suspension is then filtered and the solid is washed with 180 g of demineralized water and dried under vacuum at room temperature until constant weight, thereby obtaining 52.7 g of pure rifaximin γ having water content of 1.5%.

The form γ is characterised by a powder X-ray diffractogram showing significant peaks at diffraction angles 20 of 5.0°; 7.1 °; 8.4°.

### Example 3

### Preparation of rifaximin α

62.2 Grams of dried raw rifaximin and 163 ml of ethyl alcohol are loaded at room temperature into a three-necked flask equipped with mechanic stirrer, thermometer and reflux condenser. The suspension is heated at 57±3°C until complete dissolution of the solid and then 70 ml of demineralized water are added at this temperature during 30 minutes. After completion of the addition, the temperature is brought to 30°C during 40 minutes and is kept at this value until plentiful crystallization. The temperature of the suspension is then brought to about 40°C and kept at this value during 20 hours under stirring; then the temperature is decreased to 0°C in 30 minutes and the suspension is immediately filtered. The solid is washed with 180 ml of demineralized water and dried under vacuum at room temperature until constant weight, thereby obtaining 51.9 g of rifaximin form α with a water content equal to 2.5% and a powder X-ray diffractogram showing peaks at values of angles 2θ of 6.6°; 7.4°; 7.9°; 8.8°; 10.5°; 11.1°; 11.8°; 12.9°; 17.6°; 18.5°; 19.7°; 21.0°; 21.4°; 22.1°.

### Example 4

### Preparation of rifaximin α

89.2 Grams of raw rifaximin and 170 ml of ethyl alcohol are loaded at room temperature into a three-necked flask equipped with mechanic stirrer, thermometer and reflux condenser, then the suspension is heated at 57±3°C until complete dissolution of the solid. The temperature is brought to 50°C and then 51.7 ml of demineralized water are added at this temperature during 30 minutes. After completion of the addition the temperature is brought to 30°C in one hour and the suspension is kept for 30 minutes at this temperature obtaining a plentiful crystallization. The temperature of the suspension is brought to 40°C and kept at this value during 20 hours under stirring and then further lowered to 0°C during 30 minutes after which the suspension is immediately filtered. The solid is washed with 240 ml of demineralized water and dried under vacuum at 65°C until constant weight thereby obtaining 46.7 g of rifaximin α with a water content equal to 2.5%.

### Example 5

### Preparation of rifaximin α

Example 3 is repeated, but increasing to 50°C the temperature at which the suspension is kept and lowering to 7 hours the time in which the suspension is kept at this temperature. The product obtained is equal to that of example 3.

### Example 6

### Preparation of rifaximin β

The crystallization of the dried raw rifaximin is carried out according to the process described in example 3. Drying under vacuum at room temperature is monitored by Karl Fischer and stopped when the water content reaches 5.0%: 52.6 g of rifaximin β are obtained characterised by a powder X-ray diffractogram showing peaks at values of angles 2θ of 5.4°; 6.4°; 7.0°; 7.8°; 9.0°; 10.4°; 13.1°, 14.4°; 17.1°; 17.9°; 18.3°; 20.9°.

### Example 7

### Preparation of rifaximin α starting from rifaximin γ

5 Grams of rifaximin γ are suspended in a mixture of 13 ml of ethyl alcohol and 5.6 ml of water and the suspension is heated at 40°C during 24 hours under stirring in a 50 ml flask equipped with condenser, thermometer and mechanic stirrer. The suspension is then filtered and the solid is washed with water, then dried under vacuum at room temperature until constant weight. 4 Grams of rifaximin are obtained showing a powder X-ray diffractogram corresponding to that of the polymorphic form α and a water content equal to 2.6%.

### Example 8

### Preparation of rifaximin γ starting from rifaximin α

15 Grams of rifaximin form α and 52.4 ml of ethyl alcohol are loaded into a 250 ml three-necked flask equipped with reflux condenser, thermometer and mechanical stirrer; the suspension is heated under stirring at the temperature of 50°C until complete dissolution of the solid.

The clear solution is added with 22.5 ml of water in 30 minutes under stirring, cooled to 30°C and kept at this temperature for 30 minutes. The formed suspension is cooled to 0°C under strong stirring and kept at this temperature during 6 hours. After this time, part of the suspension is taken, filtered, washed with demineralized water and dried under vacuum at 30°C until constant weight.

The resulting product, 3.7 g, shows a diffractogram consistent with that of the form γ and a water content of 1.7%.

The remaining part of the suspension is kept at 0°C for further 18 hours under strong stirring and then is filtered, washed with demineralized water and dried at 30°C under vacuum until constant weight. 9 Grams of product showing a diffractogram consistent with that of the form γ and a water content equal to 1.6% are obtained.

### Example 9

### Preparation of rifaximin α starting from rifaximin β

5 Grams of rifaximin β having a water content equal to 5.0% are dried under vacuum at +30°C during 8 hours obtaining 4.85 g of rifaximin α having a water content equal to 2.3%.

### Example 10

### Preparation of rifaximin β starting from rifaximin α

5 Grams of rifaximin α having a water content equal to 2.5% are kept during 40 hours in an atmosphere containing a relative humidity equal to 56% made by means of a saturated aqueous solution of calcium nitrate tetrahydrate. 5.17 Grams of rifaximin β with a water content equal to 5.9% are obtained after this time.

### Example 11

### Bioavailability in dogs by oral route

Twelve 20 week pure-bred Beagle females dogs, and weighing between 5.0 and 7.5 kg, have been divided into three groups of four.

The first of these three groups has been treated with rifaximin α, the second with rifaximin β and third with rifaximin γ according to the following procedure.

Each dog received orally 100 mg/kg of one of the rifaximin polymorphs in gelatin capsules and 2 ml blood samples were collected from the jugular vein of each animal before each administration and 1, 2, 4, 6, 8 and 24 hours after the administration. Each sample was transferred into an heparinized tube and was centrifuged; the plasma was divided into 500 µl two aliquots and frozen at -20°C.

The rifaximin contained in the plasma was assayed by means of the validated LC-MS/MS method and the following parameters were calculated according to standard non-compartmental analysis:
Cₘₐₓ = maximum plasma concentration of rifaximin observed in the plasma;
Tₘₐₓ = time at which the Cₘₐₓ is reached;
AUC = area under the concentration-time curve calculated through the linear trapezoidal rule.

The results reported in the following table 2 clearly show how the rifaximin γ is very much more absorbed, more than 10² times, in respect of rifaximin α and rifaximin β which are practically not absorbed.

**Table 2**

| **Pharmacokinetic parameters for rifaximin polymorphs following single oral administration of 100 mg/kg by capsules to female dogs.** | | | |
|---|---|---|---|
| | **Cₘₐₓ ng/ml** | **tₘₐₓ h** | **AUC₀₋₂₄ ng-h /ml** |
| | **Mean** | **Mean** | **Mean** |
| **rifaximin** α | 2.632 | 9.5 | 13 |
| **rifaximin** β | 1.096 | 4 | 11 |
| **rifaximin** γ | 668.22 | 2.25 | 3908 |

### Example 12

### Intrinsic dissolution test

A sample of 100 mg of each rifaximin polymorph was submitted to the intrinsic dissolution test carried out as described in the monograph 1087 at pages 2512 - 2513 of the USP (U.S. Pharmacopoeia) 27.

100 Milligrams of a rifaximin polymorph were put into a die and compressed for 1 minute under a pressure of 5 tons by means of a punch in a hydraulic press.

A compacted pellet was formed in the die with a single face of defined area exposed on the bottom of the die so that from 50% to 75% of the compacted pellet could dissolve in an appropriate dissolution medium.

The holder containing the die was mounted on a laboratory stirring device, immersed in a glass vessel containing a dissolution medium and rotated at a rotation speed of 100 rpm by means of the stirring device, while keeping the temperature of the dissolution medium at 37± 0.5°C. The dissolution medium contained in the glass vessel consisted of 1000 ml of 0.1 M aqueous phosphate buffer pH 7.4 containing 4.5 g of sodium lauryl sulfate and was kept at 37± 0.5°C for the whole duration of the test.

Samples of 2 ml of solution were taken after 15, 30, 45 and 60 minutes from the start of the dissolution procedure and analysed by HPLC for the amount of rifaximin dissolved.

The sample containing rifaximin α systematically showed disintegration of the compacted pellet within 10 minutes and said phenomenon was also present at lower concentrations (0.1% and 0.3%) of sodium lauryl sulfate and even in absence of said surfactant, so that the value of its intrinsic dissolution could not be calculated.

The intrinsic dissolution of rifaximin γ was about ten times as much that of rifaximin β at every time, as it can be inferred by the experimental results shown in the following table 3.

**Table 3**

| **Intrinsic dissolution in 0.1 M aqueous phosphate buffer pH 7.4 with 0.45% sodium lauryl sulfate** | | |
|---|---|---|
| Time (min) | Rifaximin dissolved (mg/cm²) | |
| | β polymorph | γ polymorph |
| 15 | 0.28 | 2.46 |
| 30 | 0.50 | 4.52 |
| 45 | 0.72 | 6.44 |
| 60 | 0.94 | 9.04 |
| Intrinsic dissolution rate (mg/min/cm²) | 0.0147 | 0.1444 |

## Claims

1. Rifaximin in polymorphic form β, **characterized in that** the polymorphic form β of rifaximin is obtained by a process comprising crystallizing rifaximin from a solvent mixture by controlling the water content of the polymorph, temperature of crystallization and length of time of crystallization, wherein said β form has X-ray powder diffraction pattern peaks at about values of diffraction angles 2 θ of 5.4°; 9.0°and 20.9°.

2. A process for the production of rifaximin β comprising the steps:
- reacting a molar equivalent of rifamycin O with an excess of 2-amino-4-methylpyridine , preferably from 2.0 to 3.5 molar equivalents, in a solvent mixture of water and ethyl alcohol in volumetric ratios between 1:1 and 2:1, for a period of time between 2 and 8 hours, at a temperature between 40°C and 60°C,
- treating the reaction mass at room temperature with a solution of ascorbic acid in a mixture of water, ethyl alcohol and concentrated aqueous hydrochloric acid,
- adjusting the pH of the reaction mass to pH 2.0 with a concentrated aqueous solution of hydrochloric acid,
- filtering the suspension,
- washing any resulting solid with the water/ethyl alcohol solvent mixture to obtain raw rifaximin, purifying the raw rifaximin by dissolving it in ethyl alcohol at a temperature between 45°C and 65°|C|[MB1],
- precipitating the raw rifaximin by adding water, in weight amounts between 15% and 70%, with respect to the weight amount of ethyl alcohol used for the dissolution;
- lowering the temperature of the suspension to between 28°C and 32°C to start crystallization, keeping the resulting suspension between 40°C and 50°C under stirring for a period of time between 4 and 24 hours, and cooling to 0°C during a time between 15 minutes and one hour,
- filtering the suspension
- washing the resulting solid with water and drying it under vacuum or under conditions of normal pressure, with or without a drying agent, at a temperature between room temperature and 105°C, for a period of time between 2 and 72 hours, whereby a polymorphic form β of rifaximin having X-ray powder diffraction pattern peaks at about values of the diffraction angles 2 θ of 5.4,°; 9.0°and 20.9° is obtained.

3. A process for the production of rifaximin β comprising the steps
- dissolving raw rifaximin in ethyl alcohol at a temperature between 45°C and 65°C;
- precipitating the raw rifaximin by adding water in weight amounts between 15% and 70% with respect to the weight amount of ethyl alcohol used for dissolution;
- lowering the temperature of the suspension to between 28°C and 32°C to start crystallization, keeping the resulting suspension between 40°C and 50°C under stirring for a period of time between 4 and 24 hours and cooling to 0°C during a time between 15 minutes and 1 hour;
- filtering the suspension;
- washing the resulting solid with water and drying it under vacuum or under conditions of normal pressure, with or without drying agent, at a temperature between room temperature and 105°C, for a period of time between 2 and 72 hours, whereby a polymorphic form β or rifaximin having X-ray powder diffraction pattern peaks at about values of the diffraction angles 2 θ of 5.4°, 9.0° and 20.9° is obtained.

4. Rifaximin β prepared by a process **characterized in that** the rifaximin α having X-ray powder diffraction pattern peaks at about diffraction angles 2 θ of 27.4°,19.7°, 21.0° and 22.1° is transformed to rifaximin β by increasing the water content of the rifaximin α in amount sufficient to obtain rifaximin β having X-ray powder diffraction pattern peaks at about values of diffraction angles 2 θ of 5.4°; 9.0° and 20.9°.

5. Rifaximin β prepared by a process **characterized in that** rifaximin fi is obtained from rifaximin α having X-ray powder diffraction pattern peaks at about 2 θ values of diffraction angles of 7.4°; 19.7°; 21.0°; 22.1° by increasing the water content of polymorph α in an amount sufficient to convert the polymorph α form to the polymorph β form having X-ray powder diffraction pattern peaks at about values of diffraction angles 2 θ of 5.4°, 9.0° and 20.9°.

6. The rifaximin according to one of claims 1, 4 or 5 or obtained by a process according to one of claims 2 or 3, wherein the polymorphic form β has X-ray powder diffraction pattern peaks at about values of diffraction angles 2 θ of 5.4°; 6.4°; 7.0°; 7.8°; 9.0°; 10.4°; 13.1°; 14.4°; 17.1°; 17.9°; 18.3°; 20.9°.

7. The rifaximin according to one of claims 1, 4, 5 or 6 are obtained according to one of claims 2 or 3 for use as an antibiotic.

8. The polymorph β of rifaximin for use according to claim 7, wherein the bioavailable plasma concentration of the rifaximin reaches a maximum value (Cₘₐₓ) between about 0.0 and about 3.7 ng/ml and AUC0-24 from about 0.0 and about 40 ng·h/ml following administration of a therapeutically effective amount of the polymorph β.

9. The polymorph β of rifaximin for use according to one of claims 7 or 8 wherein the use is in a mixture of rifaximin polymorphs, wherein the amount of the rifaximin polymorphs is selected to control the amount of systemic absorption of said rifaximin.

10. The polymorph β of rifaximin for use according to claim 9 wherein the polymorphs in the mixture are polymorph α, polymorph β, and polymorph γ.

11. The polymorph β of rifaximin for use according to one of claims 7 to 10, together with pharmaceutically acceptable excipients, wherein the use is in a form of an oral or topical medicinal preparation.

12. The polymorph β of rifaximin for use according to claim 11, wherein the oral medicinal preparation is in a form selected from coated and uncoated tablets, hard and soft gelatine capsules, sugar-coated pills, lozenges, wafer sheets, pellets and powders in sealed packet.

13. The polymorph β of rifaximin for use according to claim 11, wherein the topical medicinal preparation is in a form selected from ointments, pomades, creams, gels and lotions.

14. The polymorph β optionally in admixture with polymorphs α and/or γ or rifaximin for use in a method to prevent or treat intestinal bacterial infections.

15. A medicinal preparation comprising rifaximin polymorph β according to one of claims 1, 4, 5 or 6 or obtained by a process according to one of claims 2 or 3 together with rifaximin polymorph α and rifaximin polymorph γ.

16. The medicinal preparation according to claim 15 for use as an antibiotic.
